# FASCICULE DE BREVET EUROPEEN

(11) **EP 2 585 006 B1**
(45) Date de publication et mention de la délivrance du brevet: **26.08.2015**
(21) Numéro de dépôt: 11726808.6
(22) Date de dépôt: 22.06.2011
(51) Int. Cl.: A61F 5/00, A61M 25/10

(54) **SONDE OROGASTRIQUE DE GASTRECTOMIE LONGITUDINALE**
OROGASTRISCHER KATHETER ZUR LÄNGSGASTREKTOMIE
OROGASTRIC CATHETER FOR LONGITUDINAL GASTRECTOMY

(30) Priorité: 23.06.2010 FR 1054996
(43) Date de publication de la demande: 01.05.2013
(73) Titulaire: Medical Innovation Developpement, 69570 Dardilly (FR)
(72) Inventeur: NOCCA, David, F-34000 Montpellier (FR)
(74) Mandataire: Cabinet Plasseraud
(86) Numéro de dépôt international: PCT/EP2011/060428
(87) Numéro de publication internationale: WO 2011/161148

(56) Documents cités:
- WO-A2-02/096327
- US-A- 5 401 241
- US-A1- 2005 251 158

## Description

### Domaine de l'invention

Le domaine de l'invention est celui des outils chirurgicaux utiles aux praticiens dans l'exercice de techniques de chirurgie gastrique. Les outils plus particulièrement concernés sont des sondes orogastriques visant à assister le chirurgien dans le cadre de la technique de chirurgie bariatrique de gastrectomie longitudinale ou gastrectomie en manchon, ou "sleeve gastrectomy".

### Arrière-plan technologique

Les sondes orogastriques se présentent sous la forme de tuyaux souples transparents, par exemple en silicone, ouverte à leurs deux extrémités proximale et distale. La partie distale de ces sondes comporte toute une série d'orifices latéraux permettant le passage des flux liquides et gazeux dans les deux sens. Classiquement, les sondes gastriques présentent des repères annotés sur toute leur longueur permettant d'évaluer précisément sa position in vivo.

Ces sondes peuvent être destinées à drainer le contenu de l'estomac (air, sécrétions) pour alimenter le patient, pour préciser un diagnostic, pour effectuer un lavage, ou, lors d'une opération de chirurgie bariatrique de gastrectomie longitudinale, pour drainer le contenu de l'estomac et pour aider le chirurgien à exécuter la gastrectomie longitudinale.

Cette opération chirurgicale vise à réduire la capacité gastrique (environ résection des deux tiers de l'estomac) pour provoquer un sentiment de satiété précoce et agir sur la perte pondérale.

Lors de cette intervention réalisée sous anesthésie générale et dans la grande majorité des cas par coelioscopie, une ou plusieurs sondes gastriques sont introduites par la bouche du patient et sont descendues via l'oesophage jusqu'au niveau de l'antre de l'estomac. L'intérêt de l'utilisation de ces différentes sondes est, dans l'ordre chronologique de l'intervention :
1) Exsuffler l'estomac en cas de distension aérique induite par les manoeuvres d'intubation lors de l'anesthésie.
2) Calibrer le manchon gastrique restant à l'aide du corps tubulaire de la sonde.
3) Donner des repères anatomiques au chirurgien pour réaliser une résection de qualité en évitant de sténoser la partie restante du tube gastrique (le chirurgien s'appuyant contre le corps de la sonde pour réaliser l'agrafage).
4) D'injecter puis de retirer du liquide (bleu de méthylène) dans l'estomac du patient, après l'agrafage, pour vérifier l'étanchéité de ligne d'agrafage.

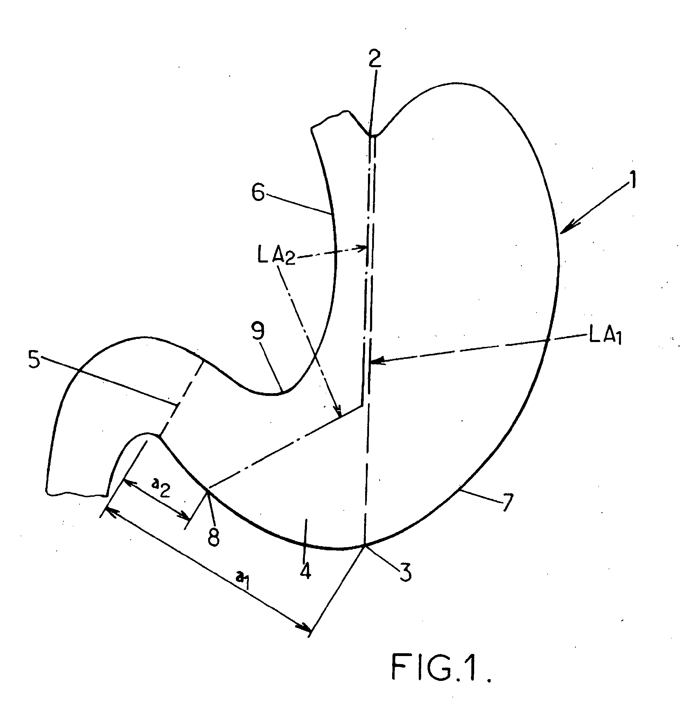

Il est à noter qu'il existe principalement deux méthodes de réalisation de l'intervention dite de "sleeve gastrectomy". La description de ces deux méthodes est faite ci-après en référence à la figure 1 annexée qui montre un schéma de l'estomac.

La première méthode, dite "avec conservation de l'antre gastrique", consiste à réaliser sur l'estomac 1 du patient par agrafage une ligne d'agrafage -LA₁- en ligne droite, de l'angle de His -2-jusqu'à un point -3- de l'antre gastrique -4- distant (a₁) de 6 à 8 cm du pylore -5-La deuxième méthode, dite "sans conservation de l'antre gastrique", consiste à réaliser une ligne d'agrafage -LA₂- d'abord concentrique à la petite courbure 6 de l'estomac 1, à partir de l'angle de His -2- jusqu'à l'angulus ou petite tubérosité (cf point de butée de l'extrémité distale de la sonde sur figure 1) -7- de l'estomac -1-, puis oblique vers un point 8 situé à 2 cm (a₂) du pylore -5- (symbolisé par un trait en pointillés sur la figure 1).

Il est connu d'utiliser une sonde gastrique de type « sonde de Salem » pour le retrait d'air présent dans l'estomac du patient (étape 1), une sonde du type « tube de Faucher » pour calibrer le manchon gastrique avant l'opération de résection-fermeture (agrafage-suture) (étapes 2 & 3), et enfin une sonde de type « sonde de Salem » pour l'injection et le retrait de liquide gastrique en fin d'intervention (étape 4). Cette dernière sonde pouvant être laissée en place quelques jours suivant les habitudes des équipes chirurgicales.

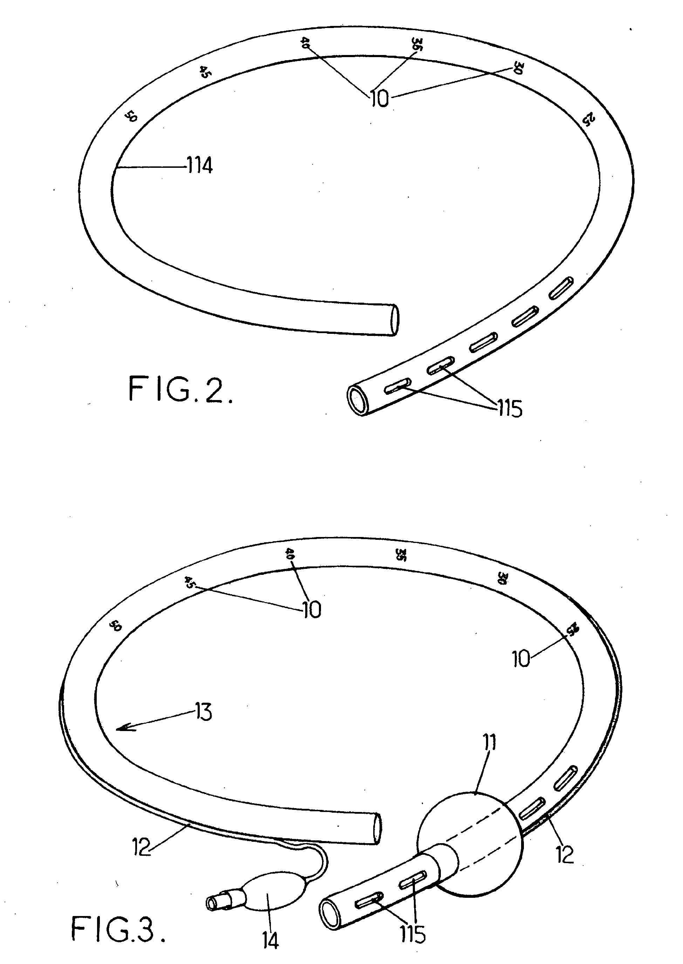

Ce « tube de Faucher » est montré sur la figure 2. Il est constitué d'un tube en silicone 114 ouvert à ses deux extrémités et dont la paroi présente des ouvertures oblongues 115 dans sa partie distale. Cette sonde est munie de repères gradués de positionnement 10.

Ce tube de Faucher est introduit dans l'estomac jusqu'à ce que son extrémité distale arrive en butée sur la grande courbure -7- de l'estomac. Le corps du tube de Faucher est en appui sur la petite courbure -6- de l'estomac. Le chirurgien a ainsi des repères anatomiques et un guide pour pouvoir réaliser une résection-fermeture (agrafage) en s'appuyant sur ce tube de Faucher 114 (étapes 2&3). Il est à noter que l'opération combinée de résection-fermeture est effectuée, de manière connue en soi, à l'aide d'un instrument chirurgical conçu pour inciser et pour fermer aussitôt par agrafage les deux bords de la plaie.

Il va de soi qu'il serait beaucoup plus commode pour les praticiens de n'avoir à mettre en oeuvre qu'une seule sonde adaptée pour toutes les étapes de l'intervention. Et ce, d'autant plus que les sondes de Salem ou les tubes de Faucher n'ont pas été spécialement conçues pour cette gastrectomie longitudinale, et souffrent donc de plusieurs inconvénients.

En particulier, ces ustensiles, usuellement en silicone, perdent leur rigidité du fait de la chaleur corporelle. Ainsi, elles ne conservent pas leur position dans l'estomac et rendent alors difficile l'opération de résection-fermeture par agrafage.

Par ailleurs, que la gastrectomie longitudinale soit effectuée, avec ou sans conservation de l'antre gastrique, les sondes de Salem ou les tubes de Faucher n'apportent aucune aide particulière, notamment au regard du calibrage de la portion d'estomac à conserver qui reste extrêmement délicate pour le chirurgien.

Un autre inconvénient des sondes classiques est qu'elles risquent d'être agrafées lors de l'intervention. En effet, ces sondes sont difficilement visibles par le chirurgien à travers la paroi de l'estomac, en coelioscopie. Il peut donc arriver que le chirurgien ne repère pas suffisamment précisément le positionnement de la sonde avant de réaliser la section de l'estomac à l'aide de son outil de résection-agrafage et qu'il agrafe cette sonde avec la paroi de l'estomac, en allant parfois jusqu'à la section complète de la sonde. Les conséquences d'un tel avatar sont potentiellement graves car le risque de fuite gastrique, avec péritonite associée, y est très important, lors de l'ablation de la sonde malencontreusement agrafée.

On connait par ailleurs une sonde orogastrique utilisée pour la pose d'anneaux gastriques. Cette sonde, représentée sur la figure 3 comporte un tube silicone 13, qui est muni de repères gradués de positionnement 10, qui est ouvert à ses deux extrémités et dont la paroi présente des ouvertures oblongues 115 dans sa partie distale. Cette dernière est également équipée d'un ballon 11 enveloppant le tube 13, c'est-à-dire que ce ballon 11 est traversé diamétralement par le tube 13. Ce ballon 11 peut être gonflé par l'intermédiaire du conduit 12 disposé à l'extérieur du tube 13 et reliant ledit ballon 11 à un moyen de gonflage 14 (soufflet fermée par une valve permettant aussi l'injection d'air) disposé dans la partie proximale de la sonde de calibration. Une fois que la partie distale de la sonde de calibration a été introduite dans l'oesophage juste en amont de l'estomac, le chirurgien procède au gonflage du ballon 11 et utilise le renflement ainsi créé comme repère pour poser l'anneau gastrique dans cette zone de raccordement de la partie abdominale de l'oesophage et l'estomac.

On connait par ailleurs US2005251158.

Dans cet état de fait, il est clair qu'il existe un besoin manifeste d'une nouvelle sonde orogastrique parfaitement adaptée pour assister le chirurgien lors d'interventions de gastrectomie longitudinale.

Le cahier des charges de cette nouvelle sonde serait qu'elle permette à elle seule :
1 - de réaliser le retrait d'air de l'estomac du patient ;
2 - de calibrer la portion d'estomac restante, quelle que soit la technique avec ou sans conservation de l'antre gastrique, pour connaître le volume exact de l'estomac restant. Le choix du type de coupe appartient alors au chirurgien lors de l'intervention ;
3 - de permettre au chirurgien de prendre des repères anatomiques et de le guider pour réaliser la section gastrique, de façon aisée, selon la technique choisie par lui, sans risque de sectionner la sonde ;
4 - de permettre l'injection ou le retrait de liquide pour vérifier l'étanchéité de la suture.

Une autre spécification de cette nouvelle sonde orogastrique serait d'avoir un corps tubulaire de forme et de rigidité adaptée à un positionnement correct et stable dans l'estomac, eu égard à la température corporelle du patient qui assouplit la sonde, pour faciliter l'opération de résection-agrafage.

Une autre attente des praticiens pour cette nouvelle sonde orogastrique serait qu'elle soit parfaitement visible pour éviter d'être agrafée lors de l'intervention.

### Problème technique - Objectifs de l'invention

Le problème technique que se propose de résoudre la présente invention est de satisfaire au moins l'un des objectifs énumérés ci-après :
- fournir une nouvelle sonde orogastrique permettant d'améliorer l'assistance procurée au chirurgien lors d'interventions de gastrectomie longitudinale ;
- fournir une nouvelle sonde orogastrique pour la gastrectomie longitudinale qui permette à elle-seule de réaliser les quatre étapes 1-4 mentionnées ci-dessus:
   exsufflage de l'estomac, calibrage de la portion d'estomac à conserver, guidage et prise de repères anatomiques, puis injection et retrait de liquide, notamment pour vérifier l'étanchéité de la ligne de suture ;
- fournir une nouvelle sonde orogastrique pour la gastrectomie longitudinale qui puisse être parfaitement calée dans l'estomac de manière à faciliter l'opération de résection-fermeture par agrafage en guidant le chirurgien ;
- fournir une nouvelle sonde orogastrique pour la gastrectomie longitudinale qui soit parfaitement repérable et visible par le chirurgien, de manière à éviter les incidents de type agrafage de la sonde ;
- fournir une nouvelle sonde orogastrique pour la gastrectomie longitudinale, qui puisse être facilement mise en place dans l'estomac, qui puisse être ainsi durablement positionnée sans bouger, sans vriller, sans changer de place et qui puisse être retirée aisément ;
- fournir une nouvelle sonde orogastrique qui permette de connaître le volume résiduel de l'estomac restant pour réaliser la résection de qualité;
- fournir une nouvelle sonde orogastrique multifonctionnelle, stérilisable;
- fournir une nouvelle sonde orograstrique pour la gastrectomie longitudinale qui soit économique et simple à fabriquer et à utiliser.

### Brève description de l'invention

Tout ou partie de ces objectifs, parmi d'autres sont atteints par la présente invention qui concerne une nouvelle sonde orogastrique conformément à la revendication 1 comprenant un corps dont la partie distale est notamment destinée, dans le cadre de la technique de chirurgie bariatrique de gastrectomie longitudinale, à guider le chirurgien pour la résection d'une partie de l'estomac et à définir la ligne de fermeture (agrafage-suture) après résection,

Cette sonde est caractérisée en ce que cette partie distale est porteuse d'un ballon présentant, à l'état gonflé, une forme sensiblement complémentaire à celle de l'antre pylorique -4-, de manière à pouvoir être logé dans cette antre pylorique -4-, l'extrémité du ballon gonflé étant alors en butée contre le pylore -5- et l'extrémité distale du corps de la sonde étant quant à elle en butée sur l'antre pylorique -4-, tandis que la partie du corps de la sonde disposée au dessus du ballon gonflé est calée contre la paroi de la petite courbure -6- de l'estomac -1-, et ainsi permettre:
- de déterminer le positionnement du début de la ligne de résection-fermeture au niveau de l'antre pylorique -4-,
- de définir la ligne de résection-fermeture,
- et de calibrer l'antre pylorique -4- et le manchon gastrique à conserver.

Cette nouvelle sonde orogastrique permet de réaliser efficacement et de manière sûre la gastrectomie longitudinale en réduisant la capacité gastrique des deux tiers et provoquer ainsi chez le patient un sentiment de satiété précoce, d'où une perte pondérale.

Cette nouvelle sonde orogastrique remédie aux difficultés passées de calibration de la portion d'estomac à éliminer. Ces difficultés pouvaient avoir comme conséquence que, faute d'avoir suffisamment réduit l'estomac, celui-ci se dilate dans le temps et prive d'effet la gastrectomie longitudinale, en laisse entier le problème de surcharge pondérale du patient.

Cette nouvelle sonde orogastrique présente donc une partie distale qui, une fois introduite dans l'estomac, peut prendre la forme de l'antre pylorique -4- par gonflage du ballon.

Cet appendice distal que forme le ballon gonflé est destiné à venir en butée sur le pylore - 5- tout en reposant sur l'antre pylorique -4- et à permettre le calage de cette partie distale de la sonde, et donc son positionnement correct et stable, en vue de la résection-fermeture de préférence par agrafage.

Cette nouvelle sonde orogastrique multifonctions améliore notablement cette technique de chirurgie bariatrique en fournissant une aide précieuse au chirurgien. L'intervention se fait plus facilement, plus rapidement, de manière plus sûre et plus efficacement quant au résultat visé.

Selon une modalité avantageuse de l'invention, les dimensions du corps de la sonde en section transversale droite, c'est-à-dire le diamètre extérieur *De* quand le corps est un tube circulaire, déterminent le volume d'estomac -1- conservé après résection, et les dimensions ou le volume du ballon gonflé, déterminent le volume d'antre pylorique -4-conservé après résection.

Pour épouser au mieux l'anatomie de la partie inférieure de l'estomac, le ballon distal gonflé a, de préférence, une forme générale (tron)conique, de préférence une forme tronconique asymétrique et distordue avec un diamètre terminal *Dt,* un diamètre à la base *Db* et un diamètre *Dm* de la partie médiane, tels que : *Dt < Db* ≤ *Dm*; une extrémité décalée par rapport à l'axe de la base du ballon, vers le haut, en direction de la partie du corps de la sonde disposée au dessus du ballon gonflé, de telle sorte que la face supérieure du ballon gonflé destinée à être disposée en regard de l'incisure angulaire -9- de l'estomac -1-, présente une courbure moins prononcée que celle de la face inférieure.

De même, pour parfaire la complémentarité de forme avec l'anatomie de l'antre pylorique - 4-, il est préférable, conformément à l'invention, que la face supérieure du ballon distal gonflé, face destinée à être disposée en regard de l'incisure angulaire -9- de l'estomac -1-, forme avec le corps de la sonde (de préférence tubulaire à section circulaire), un angle α compris entre 70 et 110°, de préférence entre 80 et 100° et, plus préférentiellement encore, de l'ordre de 90°.

Pour optimiser le calage de la partie distale de la sonde dans l'estomac -1-, il est particulièrement avantageux selon l'invention, que le ballon distal soit monté sur la face extérieure de la paroi du corps de la sonde (de préférence tubulaire à section transversale droite circulaire), de telle sorte que la distance *d* entre l'extrémité distale du corps de la sonde et le point de la base du ballon gonflé le plus proche de ladite extrémité distale, soit inférieure ou égale à 30 mm, de préférence inférieure ou égale à 20 mm, et, plus préférentiellement encore est comprise entre 1 et 15 mm.

Pour améliorer la visibilité de la sonde, il est prévu, conformément à l'invention, que le corps, de préférence tubulaire à section transversale droite, circulaire de la sonde, soit muni d'un repère permettant au chirurgien de positionner correctement le ballon dans l'estomac -1- du patient, pour qu'une fois gonflé, ce ballon puisse être logé en butée dans l'antre pylorique -4-, ce repère comprenant de préférence un trait coaxial au corps et disposé sur tout ou partie de la longueur, avantageusement toute la longueur, de la paroi du corps, du même côté que le ballon.

Avantageusement, le corps de la sonde est tubulaire et ouvert à ses deux extrémités proximale et distale, cette dernière étant de préférence équipée d'un embout de forme arrondie pour faciliter l'insertion dans la bouche et dans l'oesophage du patient.

Pour assurer sa fonction de transfert de fluides liquides ou gazeux (étapes 1 et 4 de l'intervention), entre l'intérieur et l'extérieur de l'estomac, le corps, de préférence tubulaire de la sonde, définit un canal de circulation de fluide entre l'ouverture proximale et l'ouverture distale, cette dernière étant de préférence formée par une pluralité de trous latéraux et/ou terminaux.

Pour gonfler et dégonfler, le ballon distal prévu avec la sonde selon l'invention, comporte de préférence un conduit reliant le ballon à l'extrémité proximale du corps et permettant gonflage/dégonflage dudit ballon à partir de cette extrémité proximale.

Dans un mode préféré de réalisation, la sonde selon l'invention comprend
- un corps tubulaire en silicone d'une longueur comprise entre 600 et 1200 mm, de préférence entre 700 et 1100 mm, et, plus préférentiellement encore entre 850 et 950 mm et d'un diamètre extérieur *De* compris entre 24 Fr et 75 Fr (soit entre 8 mm et 25 mm), et de préférence entre 30 Fr et 40 Fr (soit entre 10 mm et 13,33 mm), ce corps étant également pourvu dans sa partie distale, d'un embout distal arrondi et de trous latéraux destinés à mettre en communication l'intérieur de l'estomac avec l'extérieur du tube digestif par l'intermédiaire de la lumière du corps tubulaire;
- un ballon 25 gonflé de diamètre à la base *Db* comprise entre 35 et 60 mm et d'une hauteur *H* de 50 +/-10 mm, de préférence +/-5 mm, et, plus préférentiellement encore +/- 3 mm;
- un repère de positionnement formé un trait contrasté sur toute la longueur de la paroi du corps, du même côté que le ballon 25, dans le plan diamétral commun au corps et au ballon 25;
- et éventuellement des moyens de déflection de la partie distale de la sonde 20.

Idéalement, la sonde est caractérisée en ce que *H* + *De* = 50 à 100 mm, et mieux encore 60 à 80 mm.

Les moyens facultatifs de déflection de la partie distale de la sonde peuvent être prévus pour permettre de réaliser un coude sur la partie distale de la sonde, de façon à suivre l'anatomie de l'estomac -1-, dès lors que cette partie distale est introduite à l'intérieur de ce dernier. Ces moyens de déflection peuvent, par exemple, comprendre un ou plusieurs câbles de commande ou tirette(s) manoeuvrable(s) par traction et disposés le long du corps tubulaire de la sonde.
En d'autres termes, la sonde selon l'invention est conçue pour :
(i) être introduite dans la bouche du patient jusqu'à ce que son extrémité distale atteigne l'estomac du patient,
(ii) extraire des fluides l'estomac, notamment gazeux e.g. l'air en cas de distension aérique induite par les manoeuvres d'intubation lors de l'anesthésie;
(iii)gonfler le ballon et le loger dans l'antre pylorique -4- en faisant en sorte que l'extrémité du ballon gonflé soit en butée contre le pylore -5-, que l'extrémité distale du corps de la sonde soit en butée sur l'antre pylorique -4-, de préférence à une distance de 5 à 10 cm, mieux encore 6 à 8 cm du pylore -5-, et que la partie du corps de la sonde disposée au dessus du ballon gonflé soit calée contre la paroi de la petite courbure -6- de l'estomac -1-,
(iv)d'une part, guider le chirurgien pour la résection d'une partie de l'estomac -1-en déterminant le positionnement du début de la ligne de résection et de fermeture (agrafage-suture) au niveau de l'antre pylorique -4- et, d'autre part, définir la ligne de résection-fermeture, en lui donnant des repères anatomiques et un appui pour ses instruments lors de ces actes de résection-fermeture,
(v) calibrer le manchon gastrique et l'antre pylorique -4- à conserver,
(vi)injecter puis retirer du liquide coloré dans l'estomac -1-du patient, après la fermeture, pour vérifier l'étanchéité de ligne de fermeture (suture),
(vii) et éventuellement introduire au sein de l'estomac -1- des moyens de visualisation (source lumineuse).

Un exemple concerne également une méthode chirurgicale de gastrectomie longitudinale (ou "sleeve gastrectomy") consistant à mettre en oeuvre les étapes (i) à (vii) susdécrites.

L'introduction de moyens de visualisation, comme une fibre optique, peut être réalisée à tout moment de l'intervention, et de préférence dès le début de celle-ci, en utilisant un canal coaxial prévu dans la paroi ou dans la lumière du corps tubulaire de la sonde.

### Description détaillée de l'invention

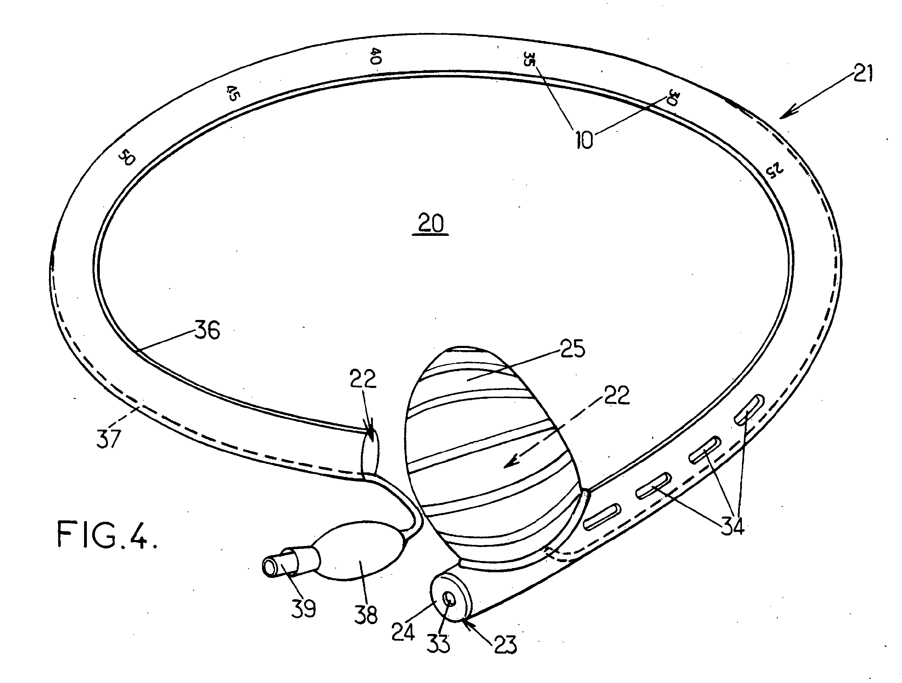

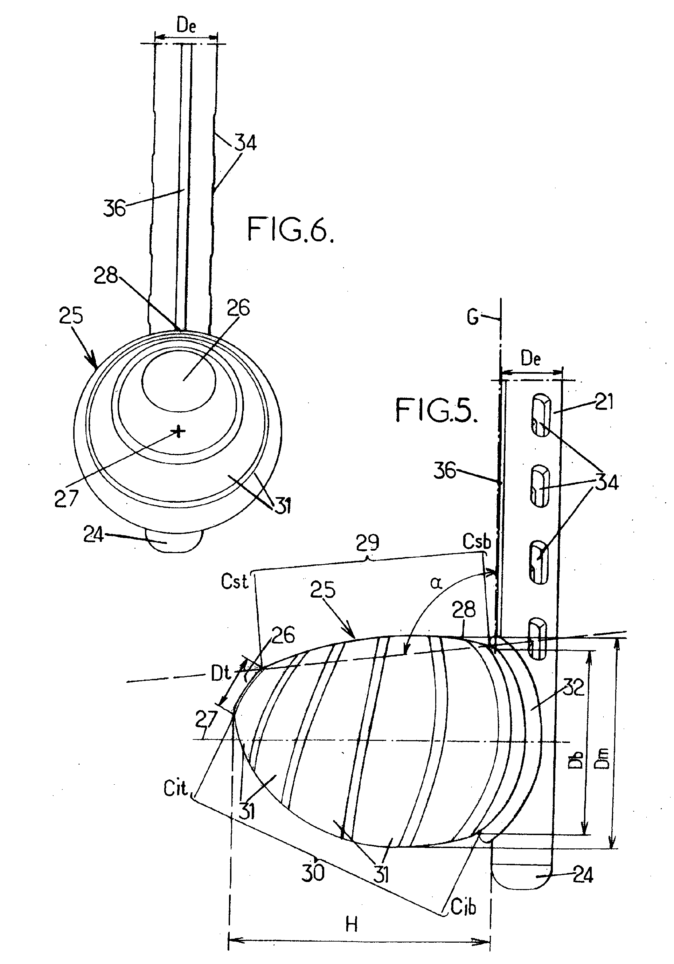

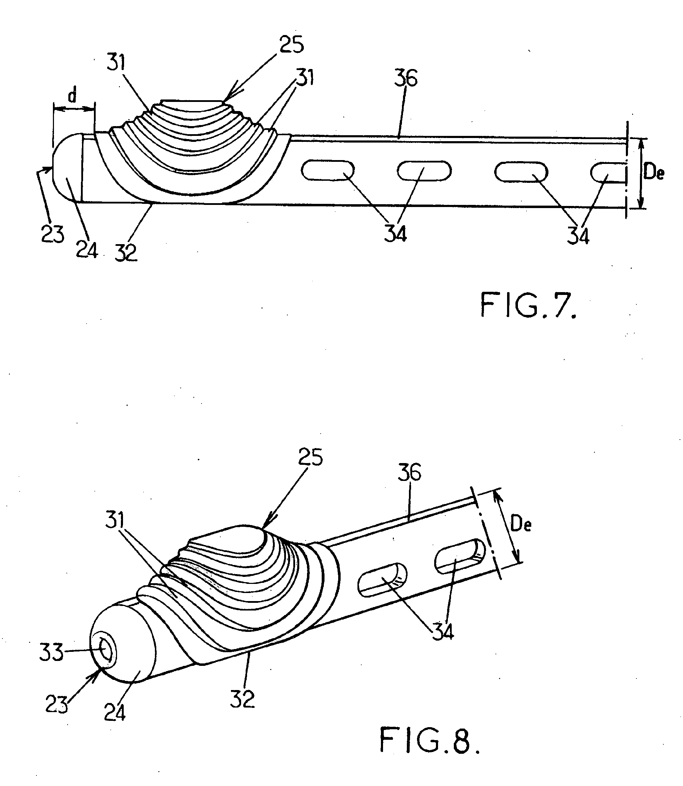

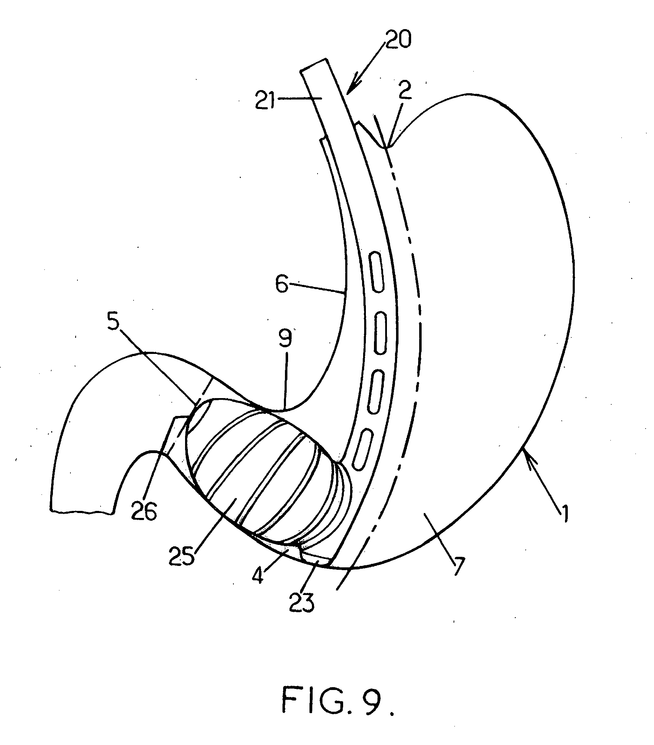

L'invention décrite ci-après est un exemple de réalisation de la nouvelle sonde orogastrique selon l'invention, en référence aux dessins annexés dans lesquels :
- **les** **figures 1 à 3** représentent respectivement l'anatomie de l'estomac et deux éléments de l'art antérieur ;
- **la** **figure 4** générale présente la nouvelle sonde orogastrique formée par un corps tubulaire de section circulaire en silicone, dont l'extrémité distale est équipée d'un ballon gonflé, destiné à venir se loger dans l'antre pylorique ;
- **la** **figure 5** est une vue de côté et de détail de la partie distale de la sonde sur laquelle est monté le ballon gonflé ;
- **la** **figure 6** est une vue de face de la figure 5 ;
- **la** **figure 7** est une vue de côté de la partie distale de la sonde avec le ballon à l'état dégonflé ;
- **la** **figure 8** est un agrandissement de la figure 7 selon une vue en perspective de ¾ avant ;
- **la** **figure 9** est un schéma montrant l'implantation de la sonde dans l'estomac.

La sonde orogastrique conformément à la revendication 1 montrée dans son ensemble sur la figure 4 est désignée par la référence générale 20 comprend un corps tubulaire 21 en silicone de section transversale droite circulaire. Ce corps tubulaire 21 présente une extrémité proximale ouverte 22 et une extrémité distale 23, munie d'un embout distal 24. Le diamètre extérieur *De* montré sur les figures 5 à 8 de ce corps tubulaire 21 est par exemple de 12,5 mm, soit 37.5 Fr (French bougies). Ce diamètre extérieur *De* calibre le volume de l'estomac -1- à conserver. Ainsi, plusieurs diamètres *De* de ce corps tubulaire 21 et donc plusieurs calibres peuvent être proposés au chirurgien.
La longueur totale du corps tubulaire 21 depuis l'extrémité proximale 22 jusqu'à l'extrémité distale 23 est, par exemple, de 900 mm.
Le corps tubulaire 21 a avantageusement une longueur totale suffisante pour que sa partie distale, en particulier le ballon 25 gonflé puisse être placé dans l'antre pylorique-4- de l'estomac -1- (cf. figures 1 et 9). Le corps tubulaire 21 est de préférence en élastomère silicone transparent. Sa structure, ses caractéristiques mécaniques dont notamment son élasticité, sont choisis de manière à ce que la rigidité de la sonde soit suffisante pour qu'elle puisse être introduite par la bouche du patient jusqu'à l'estomac, en passant par l'oesophage, sans endommager les parois du tube digestif, tout en conservant une forme sensiblement rectiligne pour épouser la morphologie anatomique de l'estomac -1-, du patient, en particulier de la petite courbure gastrique -6- s'agissant du corps tubulaire 21 et l'antre pylorique -4- s'agissant du ballon 25 gonflé. Ainsi, le tube e.g. en élastomère silicone formant le corps de la sonde, celui-ci a avantageusement une dureté SHORE de 65 ±10.

Avantageusement, le corps tubulaire 21 de la sonde 20 est gradué de 5 cm en 5 cm (graduations 10) à partir de l'extrémité distale 23.

Par ailleurs la partie distale du tube 21 de la sonde 20 est porteuse d'un ballon 25 présentant à l'état gonflé une forme sensiblement complémentaire à celle de l'antre pylorique -4-. Ce ballon 25 est monté sur la face extérieure (face anatomique droite--figure 9-) de la paroi du corps tubulaire 21, de telle sorte que le plan longitudinal médian du ballon 25 gonflé, corresponde sensiblement au plan longitudinal médian du corps tubulaire 21. Ces plans longitudinaux médians du ballon gonflé 25 et du tube 21 équivalent sensiblement au plan frontal de coupe anatomique (figure 9).
Ce ballon gonflé 25 a une forme générale tronconique asymétrique et distordue avec, d'une part, un diamètre terminal *Dt,* un diamètre à la base *Db,* et un diamètre *Dm* de la partie médiane telle que *Dt < Db* ≤ *Dm,* et, d'autre part, une extrémité 26 décalée, par rapport à l'axe 27 de la base du ballon gonflé 25, vers le haut, en direction de la partie du corps tubulaire 21 de la sonde 20 disposée au dessus du ballon gonflé 25, de telle sorte que la face supérieure du ballon gonflé 25 destinée à être disposée en regard de l'incisure angulaire -9- de l'estomac -1-, présente une courbure supérieure 29 {entre les points *Cst* et *Csb* de la figure 5} de la face supérieure 28, moins prononcée que la courbure inférieure 30 {entre les points *Cit* et *Cib* de la figure 5} de la face inférieure. Le rayon de courbure de la courbure supérieure 29 (*Cst-Csb*) est supérieur à celui de la courbure inférieure 30 (*Cit-Cib*).
La hauteur *H* du ballon 25 gonflé est mesurée dans le plan longitudinal médian du corps tubulaire 21 et du ballon 25 gonflé, à partir du bord du corps tubulaire 21 situé juste en amont de l'embout distal 24 et en aval du ballon 25 (côté droit de l'estomac), jusqu'au sommet du ballon 25 gonflé (voir figure 5).
Par exemple : *Db* = 40 mm; *H* = 50 mm ; *Dm* = 50mm et *Dt* = 10mm.
En d'autres termes, les dimensions et l'élasticité du ballon 25 sont telles que le volume du ballon gonflé peut par exemple être de 75 cm³ +/- 25 cm³.
Suivant une autre caractéristique de l'invention, la face supérieure 28 du ballon gonflé 25 destiné à être disposé en regard de l'incisure angulaire -9- (cf. figures 1 et 9) de l'estomac -1- forme avec le corps tubulaire 21 de la sonde 20, un angle α égal, dans cet exemple, à 90° environ (cf. figure 5).
Comme cela ressort de cette figure 5, l'angle α est défini par la droite passant par les deux points Csb et Cst du ballon (25) gonflé et par le corps tubulaire 21- génératrice G.
Ce ballon 25 est constitué par une membrane, par exemple en élastomère silicone de préférence faite de plusieurs segments annulaires 31 superposés, solidaires les uns des autres, et de dimensions choisies pour donner au ballon gonflé 25, la forme décrite ci-dessus et montrée sur les figures 4, 5, 6 et 9.
La base du ballon 25 présente une lèvre 32 soudée et/ou collée sur le tube 21 de la sonde 20. Comme cela apparaît sur les figures 7 et 8, la base 32 du ballon 25 enveloppe le tube 21 sur une grande partie de sa circonférence.

Par ailleurs, comme montré sur la figure 7, la distance *d* entre, d'une part, l'extrémité distale du tube 21 correspondant à l'extrémité distale 23 de l'embout 24 et, d'autre part, le point de la base du ballon gonflé 25 le plus proche de cette extrémité distale 23, est, par exemple, en l'espèce de 10 mm. Il est avantageux que *d* soit le plus faible possible de manière à s'adapter au mieux à la forme anatomique de l'antre pylorique -4-.
Cet embout distal arrondi 24 est percé en son centre d'un trou 33 mettant en communication la lumière du tube 21 avec l'extérieur, de la même façon que les trous latéraux oblongs 34 présents dans la partie distale du tube 21, en amont du ballon 25. Ces orifices 33, 34 permettent le retrait et l'injection de fluides gazeux ou liquides de l'estomac.

Selon l'invention, le calibrage de la partie d'estomac à conserver n'est pas seulement assuré par le diamètre extérieur *De* du corps tubulaire 21, mais aussi par le volume du ballon 25 gonflé. Plusieurs volumes de gonflage du ballon 25 donnent autant de calibre au chirurgien.

La sonde 20 est également munie d'un repère 36 de positionnement. Ce repère 36 est formé par un trait contrasté sur toute la longueur de la paroi du tube 21 de l'extrémité proximale 22 à l'extrémité distale 23. Il est perpendiculaire à l'axe 27 du ballon gonflé 25, dans le plan longitudinal médian commun audit ballon gonflé 25 et au tube 21. Il est placé du côté droit de l'estomac, c'est-à-dire du côté où le ballon 25 peut se gonfler dans l'antre pylorique -4-. Il indique notamment au chirurgien la face extérieure de la paroi du tube 21 sur laquelle est monté le ballon 25 (côté anatomique droit sur la figure 9). Cette face vient alors en butée sur la petite courbure. La mise en place du ballon gonflé 25 dans l'antre pylorique -4- est ainsi grandement facilitée par ce repère contrasté 36.

La sonde 20 selon l'invention est également équipée d'un conduit 37 reliant le ballon 25 à l'extrémité proximale 22 du corps tubulaire 21 et permettant le gonflage/dégonflage dudit ballon 25 à partir de cette extrémité proximale, par l'intermédiaire d'un moyen de gonflage qui peut être par exemple un ballonnet 38 prévu à l'extrémité proximale 37 reliée à l'extérieur par l'intermédiaire d'une valve 39, et/ou un injecteur de fluide (e.g. air) telle qu'une seringue qui peut être introduite dans l'embout du conduit 37 en amont de la valve 39. Le conduit de gonflage 37 du ballon 25 chemine dans l'épaisseur de la paroi du corps tubulaire 21 du même côté que le repère de positionnement 36 ou de l'autre côté. L'extrémité distale du conduit de gonflage 37 débouche à l'intérieur du ballon distal 25.

La figure 9 montre le positionnement stable de la partie distale du corps tubulaire 21 contre la petite courbure -6- et le blocage du ballonnet 25 gonflé dans l'antre pylorique -4-. Le positionnement du ballon gonflé 25 et de la partie distale du corps tubulaire 21 montré sur la figure 9, permet au chirurgien d'avoir des repères anatomiques et un guide pour procéder à la résection-agrafage de la partie gauche de l'estomac -1- à éliminer (trait mixte sur la figure 9), suivant le calibrage également défini par le ballonnet gonflé et la partie distale du tube 21 en appui contre la petite courbure -6-.
Les moyens de déflection facultatifs susceptibles de permettre le pliage de la partie distale de la sonde, par exemple 88 mm à partie de l'extrémité distale, d'un angle e.g. d'environ 90°, comprennent un système de câble ou de tirette inséré dans un canal coaxial au tube 21 du côté droit de l'estomac, là où est situé le ballon 25. Le câble descend dans ce canal pour être fixé dans la partie distale de la sonde. Au-dessus du point de fixation du câble, de l'embout distal 24 de la sonde 20, une entaille d'un angle d'environ 45° est faite dans le corps 21 de la sonde 20 de manière à créer une ligne de pliure. Une membrane est collée sur l'entaille pour assurer l'étanchéité du corps de la sonde. Une fois la sonde en place dans l'estomac -1- du patient, le câble peut être tendu par l'action de la main du chirurgien au niveau de la partie proximale de la sonde, et cette action a pour conséquence d'entraîner le pliage ou la déflection de la partie distale terminale du corps tubulaire 21 qui forme un angle, par exemple de 90° avec la partie supérieure dudit corps tubulaire 21.

La lumière du corps tubulaire 21 peut permettre l'introduction de moyens de visualisation tels qu'une source lumineuse, par exemple une fibre optique souple.

Il est possible de prévoir un traitement de surface de la face externe du corps tubulaire 21 et du ballon 25, de manière à réduire le coefficient de friction et ainsi faciliter le glissement de la sonde 20 contre les tissus internes du tube digestif du patient.

Comme l'illustre la figure 9, cette sonde orogastrique 20 peut être introduite, en préalable à la gastrectomie longitudinale, dans la bouche du patient jusqu'à ce que son extrémité distale 23 atteigne l'estomac -1-. La suite de la gastrectomie longitudinale consiste alors à exsuffler l'estomac en cas de distension aérique induite par les manoeuvres d'intubation lors de l'anesthésie, puis à gonfler le ballon 25 et à le loger dans l'antre pylorique -4-, en faisant en sorte que:
- l'extrémité 26 du ballon gonflé 25 soit par exemple en butée contre le pylore -5- (symbolisé en traits pointillés sur la figure 9), que l'extrémité distale 23 du corps tubulaire 21 soit par exemple en butée sur l'antre pylorique -4-, par exemple à environ 5 à 10 cm du pylore -5-,
- et que la partie du corps tubulaire 21 de la sonde 20 disposée au-dessus du ballon gonflé 25 soit calée contre la paroi de la petite courbure de l'estomac 6.

Le chirurgien peut ensuite procéder, à l'aide d'un outil approprié, à une résection-agrafage de la partie gauche de l'estomac (trait mixte sur la figure 9) en étant guidé par le ballon 25 gonflé et la partie distale du tube 21, qui lui indique le début de la ligne de résection/fermeture et cette ligne de résection-fermeture elle-même, en fournissant également, en plus de ces repères anatomiques, un appui pour son instrument de résection-fermeture. Le ballon 25 gonflé et la partie distale du tube 21 calibre aussi le manchon gastrique et l'antre pylorique 4 à conserver.
Après résection de la partie gauche et agrafage, il est possible d'injecter du liquide coloré dans l'estomac 1 du patient pour vérifier l'étanchéité de la ligne de fermeture.

La source lumineuse permettant de visualiser l'intérieur de l'estomac peut être introduite dans le corps tubulaire 21 à un moment quelconque de l'intervention.

Le chirurgien dispose ainsi d'une sonde orogastrique multifonction lui permettant de réaliser rapidement une gastrectomie longitudinale de qualité et en toute sécurité pour le patient.

## Revendications

1. Sonde (20) orogastrique comprenant un corps (21) dont la partie distale est notamment destinée, dans le cadre de la technique de chirurgie bariatrique de gastrectomie longitudinale, à guider le chirurgien pour la résection d'une partie de l'estomac et à définir la ligne de fermeture après résection, telle partie distale est porteuse d'un ballon (25) présentant, à l'état gonflé, une forme sensiblement complémentaire à celle de l'antre pylorique (4), de manière à pouvoir être logé dans cette antre pylorique (4), l'extrémité (26) du ballon (25) gonflé étant alors adaptée à être en butée contre le pylore tandis que la partie du corps (21) de la sonde (20) disposée au dessus du ballon (25) gonflé est adaptée à être calée contre la paroi de la petite courbure (6) de l'estomac (1), (5) la sonde étant **caractérisée en ce que** l'extrémité distale du corps de la sonde (20) est quant à elle adaptée à être en butée sur l'antre pylorique (4), de manière à pouvoir ainsi permettre:
- de déterminer le positionnement du début de la ligne de résection-fermeture au niveau de l'antre pylorique (4),
- de définir la ligne de résection-fermeture,
- et de calibrer l'antre pylorique (4) et le manchon gastrique à conserver.

2. Sonde (20) selon la revendication 1, **caractérisée en ce que** les dimensions du ballon (25) gonflé en section transversale droite, déterminent le volume de l'antre pylorique (4) conservé après résection et **en ce que** les dimensions du corps (21) en section transversale droite déterminent le volume d'estomac (1) conservé après résection.

3. Sonde (20) selon la revendication 1 ou 2, **caractérisée en ce que** le ballon (25) gonflé a une forme générale (tron)conique, de préférence une forme tronconique asymétrique et distordue avec un diamètre terminal *Dt,* un diamètre à la base *Db* et un diamètre *Dm* de la partie médiane, tels que : *Dt < Db* ≤ *Dm;* une extrémité décalée par rapport à l'axe de la base du ballon (25), vers le haut, en direction de la partie du corps de la sonde (20) disposée au dessus du ballon (25) gonflé, de telle sorte que la face supérieure du ballon (25) gonflé destinée à être disposée en regard de l'incisure angulaire de l'estomac, présente une courbure moins prononcée que celle de la face inférieure.

4. Sonde (20) selon l'une au moins des revendications précédentes, **caractérisée en ce que** la face supérieure (28) du ballon (25) gonflé destinée à être disposée en regard de l'incisure angulaire (9) de l'estomac (1), forme avec le corps (21) de la sonde (20) un angle α compris entre 70 et 110° de préférence entre 80 et 100°, et, plus préférentiellement encore de l'ordre de 90°.

5. Sonde (20) selon la revendication 1 ou 2 **caractérisée en ce que** le ballon (25) est monté sur la face extérieure de la paroi du corps de la sonde (20), de telle sorte que la distance d entre l'extrémité distale (23) du corps (21) de la sonde (20) et le point de la base du ballon (25) gonflé le plus proche de ladite extrémité distale (23), soit inférieure ou égale à 30 mm, de préférence inférieure ou égale à 20 mm, et, plus préférentiellement encore, est comprise entre 1 et 15 mm.

6. Sonde (20) selon l'une au moins des revendications précédentes, **caractérisée en ce que** le corps (21) est muni d'un repère (36) permettant au chirurgien de positionner correctement le ballon (25) dans l'estomac du patient, pour qu'une fois gonflé, ce ballon (25) puisse être logé en butée dans l'antre pylorique (4), ce repère (36) comprenant de préférence un trait coaxial au corps et disposé sur tout ou partie de la longueur, avantageusement toute la longueur, de la paroi du corps (21), du même côté que le ballon (25).

7. Sonde (20) selon l'une au moins des revendications précédentes, **caractérisée en ce qu'**elle comporte un conduit reliant le ballon (25) à l'extrémité proximale du corps et permettant gonflage/dégonflage dudit ballon (25) à partir de cette extrémité proximale.

8. Sonde (20) selon l'une au moins des revendications précédentes, **caractérisée en ce qu'**elle comprend:
• un corps tubulaire en silicone d'une longueur comprise entre 600 et 1200 mm, de préférence entre 700 et 1100 mm, et, plus préférentiellement encore entre 850 et 950 mm et d'un diamètre extérieur *De* compris entre 24 Fr et 75 Fr (soit entre 8 mm et 25 mm), et de préférence entre 30 Fr et 40 Fr (soit entre 10 mm et 13,33 mm), ce corps étant également pourvu dans sa partie distale, d'un embout distal arrondi et de trous latéraux destinés à mettre en communication l'intérieur de l'estomac avec l'extérieur du tube digestif par l'intermédiaire de la lumière du corps tubulaire;
• un ballon (25) gonflé de diamètre à la base *Db* comprise entre 35 et 60 mm et d'une hauteur *H* de 50 +/-10 mm, de préférence +/-5 mm, et, plus préférentiellement encore +/- 3 mm;
• un repère de positionnement formé un trait contrasté sur toute la longueur de la paroi du corps, du même côté que le ballon (25), dans le plan diamétral commun au corps et au ballon (25);
• et éventuellement des moyens de déflection de la partie distale de la sonde (20).

9. Sonde (20) selon la revendication 8, **caractérisée en ce que** *H* + *De* = 50 à 100 mm, mieux encore 60 à 80 mm.

## Patentansprüche

1. Orogastrischer Katheter (20), der einen Körper (21) umfasst, dessen distaler Teil insbesondere vorgesehen ist, im Rahmen der Adipositas-Chirurgie der Längsgastrektomie den Chirurgen bei der Resektion eines Teils des Magens zu führen und die Verschlusslinie nach der Resektion festzulegen, jener distaler Teil einen Ballon (25) trägt, der im aufgepumpten Zustand eine Form aufweist, die praktisch komplementär zu derjenigen des Antrum pyloricum (4) ist, um in dieses Antrum pyloricum (4) aufgenommen werden zu können, wobei das Ende (26) des aufgepumpten Ballons (25) dann so angepasst ist, dass es gegen den Pylorus (5) anliegt, während der Teil des Körpers (21) des Katheters (20), der über dem aufgeblasenen Ballon (25) angeordnet ist, so angepasst ist, dass er gegen die Wand der kleinen Kurvatur (6) des Magens (1) gelehnt werden kann, wobei der Katheter **dadurch gekennzeichnet ist, dass**
das distale Ende des Körpers des Katheters (20) selbst derart angepasst ist, dass er so gegen das Antrum pyloricum (4) anliegt, dass er auf diese Weise Folgendes ermöglichen kann:
- die Positionierung des Anfangs der Resektions-Verschlusslinie auf der Ebene des Antrum pyloricum (4) zu bestimmen,
- die Resektions-Verschlusslinie festzulegen,
- und das Antrum pyloricum (4) sowie die zu erhaltende Magen-Sleeve zu kalibrieren.

2. Katheter (20) nach Anspruch 1, **dadurch gekennzeichnet, dass** die Abmessungen des aufgepumpten Ballons (25) in geradem Querschnitt das nach der Resektion beibehaltene Volumen des Antrum pyloricum (4) bestimmen und dass die Abmessungen des Körpers (21) in geradem Querschnitt das nach der Resektion beibehaltene Volumen des Magens (1) bestimmen.

3. Katheter (20) nach Anspruch 1 oder 2, **dadurch gekennzeichnet, dass** der aufgepumpte Ballon (25) eine im Allgemeinen kegel(stumpf)förmige Form, vorzugsweise eine asymmetrische und gekrümmte kegelstumpfförmige Form mit einem terminalen Durchmesser *Dt,* einem Durchmesser an der Basis *Db* und einem Durchmesser *Dm* des Mittelteils, so dass *Dt < Db* ≤ *Dm;* ein Ende, welches im Verhältnis zu der Achse der Basis des Ballons (25) derart nach oben versetzt ist, in Richtung des Teils des Körpers des Katheters (20), der über dem aufgepumpten Ballon (25) angeordnet ist, dass die Oberseite des aufgepumpten Ballons (25), die vorgesehen ist, der winkligen Inzision des Magens gegenüber liegend angeordnet zu werden, eine weniger ausgeprägte Krümmung als die Unterseite aufweist.

4. Katheter (20) nach mindestens einem der vorangegangenen Ansprüche, **dadurch gekennzeichnet, dass** die Oberseite (28) des aufgepumpten Ballons (25), die vorgesehen ist, der winkligen Inzision (9) des Magens (1) gegenüber liegend angeordnet zu werden, mit dem Körper (21) des Katheters (20) einen Winkel α zwischen einschließlich 70 und 110°, vorzugsweise zwischen 80 und 100°, und noch bevorzugter in der Größenordnung von 90° bildet.

5. Katheter (20) nach Anspruch 1 oder 2 **dadurch gekennzeichnet, dass** der Ballon (25) auf der Außenseite der Wand des Körpers des Katheters (20) derart angebracht ist, dass die Distanz *d* zwischen dem distalen Ende (23) des Körpers (21) des Katheters (20) und dem am nächsten zu dem distalen Ende (23) liegenden Punkt der Basis des aufgeblasenen Ballons (25) kleiner oder gleich 30 mm, vorzugsweise kleiner oder gleich 20 mm und noch bevorzugter zwischen einschließlich 1 und 15 mm ist.

6. Katheter (20) nach mindestens einem der vorangegangenen Ansprüche, **dadurch gekennzeichnet, dass** der Körper (21) mit einer Kennzeichnung (36) versehen ist, die es dem Chirurgen ermöglicht, den Ballon (25) in dem Magen des Patienten richtig zu positionieren, damit dieser Ballon (25), wenn er aufgepumpt ist, im Antrum pyloricum (4) anliegend aufgenommen werden kann, wobei die Kennzeichnung (36) vorzugsweise ein zum Körper koaxiales Merkmal umfasst und über die Gesamtheit oder einen Teil der Länge, vorteilhafterweise die gesamte Länge der Wand des Körpers (21) auf derselben Seite des Ballons (25) angeordnet ist.

7. Katheter (20) nach mindestens einem der vorangegangenen Ansprüche, **dadurch gekennzeichnet, dass** er eine den Ballon (25) mit dem proximalen Ende des Körpers verbindende Leitung umfasst, die das Aufpumpen/Abschwellen des Ballons (25) von dem proximalen Ende aus ermöglicht.

8. Katheter (20) nach mindestens einem der vorangegangenen Ansprüche, **dadurch gekennzeichnet, dass** er Folgendes umfasst:
- einen rohrförmigen Körper aus Silikon mit einer Länge zwischen einschließlich 600 und 1200 mm, vorzugsweise zwischen 700 und 1100 mm und noch bevorzugter zwischen 850 und 950 mm, und einem Außendurchmesser *De* zwischen einschließlich 24 Fr und 75 Fr (d. h. zwischen 8 mm und 25 mm), und vorzugsweise zwischen 30 Fr und 40 Fr (d. h. zwischen 10 mm und 13,33 mm), wobei der Körper außerdem in seinem distalen Teil mit einem distalen abgerundeten Ende und seitlichen Löchern versehen ist, die dazu dienen, das Innere des Magens mit dem Äußeren des Verdauungskanals mittels des Lumens des rohrförmigen Körpers in Verbindung zu bringen;
- einen aufgepumpten Ballon (25) mit einem Durchmesser an der Basis *Db* zwischen einschließlich 35 und 60 mm und einer Höhe *H* von 50 +/-10 mm, vorzugsweise +/-5 mm und noch bevorzugter +/-3 mm;
- eine Positionierungskennzeichnung, die ein kontrastreiches Merkmal über die gesamte Länge der Wand des Körpers auf derselben Seite wie der Ballon (25) auf der gemeinsamen diametralen Ebene des Körpers und des Ballons (25) bildet;
- und gegebenenfalls Mittel zur Ablenkung des distalen Teils des Katheters (20).

9. Katheter (20) nach Anspruch 8, **dadurch gekennzeichnet, dass** *H* + *De =* 50 bis 100 mm, noch besser 60 bis 80 mm.

## Claims

1. Orogastric catheter (20) comprising a body (21) the distal part of which is in particular intended, in the context of the bariatric surgical technique of longitudinal gastrectomy, to guide the surgeon for resection of a part of the stomach and to define the closure line after resection, said distal part carrying a balloon (25) having, in the inflated state, a shape substantially matching that of the pyloric antrum (4), in such a way that it can be housed in said pyloric antrum (4), the end (26) of the inflated balloon (25) then being adapted to be in abutment against the pylorus (5) whereas the part of the body (21) of the catheter (20) arranged above the inflated balloon (25) is adapted to be wedged against the wall of the lesser curvature (6) of the stomach (1),
The catheter being **characterized in that** the distal end of the body of the catheter (20) is for its part adapted to be in abutment against the pyloric antrum (4) so as to thus permit:
- determining the position of the start of the closure resection line in the area of the pyloric antrum (4),
- defining the closure resection line,
- and calibrating the pyloric antrum (4) and the gastric sleeve to be preserved.

2. Catheter (20) according to claim 1, **characterized in that** the dimensions of the inflated balloon (25) in cross-section determine the volume of the pyloric antrum (4) preserved after resection and **in that** the dimensions of the body (21) in cross-section determine the volume of stomach (1) preserved after resection.

3. Catheter (20) according to claim 1 or 2, **characterized in that** the inflated balloon (25) has a (truncated) conical general shape, preferably a truncated conical shape that is asymmetric and distorted with a terminal diameter *Dt,* a diameter at the base *Db* and a diameter *Dm* of the middle part, such that: *Dt < Db* ≤ *Dm;* an end that is offset relative to the axis of the base of the balloon (25), upwards towards the part of the body of the catheter (20) arranged above the inflated balloon (25), so that the upper face of the inflated balloon (25), intended to be arranged opposite the angular notch of the stomach, has a less pronounced curvature than that of the lower face.

4. Catheter (20) according to at least one of the preceding claims, **characterized in that** the upper face (28) of the inflated balloon (25) intended to be arranged opposite the angular notch (9) of the stomach (1) makes, with the body (21) of the catheter (20), an angle α between 70 and 110°, preferably between 80 and 100°, and even more preferably of the order of 90°.

5. Catheter (20) according to claim 1 or 2, **characterized in that** the balloon (25) is mounted on the outer face of the wall of the catheter body (20), in such a way that the distance d between the distal end (23) of the body (21) of the catheter (20) and the point of the base of the inflated balloon (25) closest to said distal end (23), is less than or equal to 30 mm, preferably less than or equal to 20 mm, and even more preferably is between 1 and 15 mm.

6. Catheter (20) according to at least one of the preceding claims, **characterized in that** the body (21) is provided with a reference mark (36) enabling the surgeon to position the balloon correctly (25) in the patient's stomach, so that once inflated, this balloon (25) can be lodged in abutment in the pyloric antrum (4), said reference mark (36) preferably comprising a mark coaxial to the body and arranged on some or all of the length, advantageously the entire length, of the wall of the body (21), on the same side as the balloon (25).

7. Catheter (20) according to at least one of the preceding claims, **characterized in that** it comprises a tube connecting the balloon (25) to the proximal end of the body and allowing inflation/deflation of said balloon (25) from this proximal end.

8. Catheter (20) according to at least one of the preceding claims, **characterized in that** it comprises:
• a tubular body of silicone with a length between 600 and 1200 mm, preferably between 700 and 1100 mm, and even more preferably between 850 and 950 mm and with an outside diameter *De* between 24 Fr and 75 Fr (i.e. between 8 mm and 25 mm), and preferably between 30 Fr and 40 Fr (i.e. between 10 mm and 13.33 mm), said body also being provided in its distal part with a rounded distal tip and lateral holes intended to provide communication between the interior of the stomach and the exterior of the alimentary canal via the lumen of the tubular body;
• an inflated balloon (25) with a diameter at the base *Db* between 35 and 60 mm and with a height *H* of 50 +/- 10 mm, preferably +/-5 mm, and even more preferably +/- 3 mm;
• a positioning reference mark formed by a contrasting mark on the entire length of the wall of the body, on the same side as the balloon (25), in the diametrical plane common to the body and to the balloon (25);
• and optionally means for deflecting the distal part of the catheter (20).

9. Catheter (20) according to claim 8, **characterized in that** *H* + *De =* 50 to 100 mm, even better 60 to 80 mm.
